# EUROPEAN PATENT APPLICATION

(11) **EP 1 568 786 A2**
(43) Date of publication of application: **31.08.2005**
(21) Application number: 05250838.9
(22) Date of filing: 14.02.2005
(51) Int. Cl.: C12Q 1/68

(54) **Analysis of methylation status using nucleic acid arrays**

(30) Priority: 13.02.2004 US 544844 P; 03.12.2004 US 633062 P
(71) Applicant: Affymetrix, Inc. (A US Entity), California 95051 (US)
(72) Inventor: Lipschultz, Robert, J., Palo Alto, CA 94301 (US); Fodor, Stephen, P., A., Palo Alto, CA 94301 (US); Nautiyal, Shivani, San Fransisco, CA 940132 (US)
(74) Representative: Bizley, Richard Edward

(57) **Abstract**

Methods for amplifying a nucleic acid sample while preserving the methylation status of cytosines are disclosed. In some aspects the amplified methylated sample is modified by methylation sensitive modification and analyzed by hybridization to an array to identify cytosines that were methylated in the starting material and cytosines that were not methylated in the starting material. Methods for detecting methylation status are also disclosed. In one embodiment a DNA methyltransferase activity is included in the amplification reaction and this activity methylates the newly synthesized DNA using the methylated genomic template strand as a guide. Other aspects concern an array of probes which are complementary to restriction sites and methods for analysing methylation employing restriction enzymes and adaptors.

## Description

### RELATED APPLICATIONS

This application claims the benefit of U.S. Provisional Application Nos. 60/544,844 filed February 13, 2004 and 60/633,062 filed December 3, 2004. The entire disclosure of the above applications is incorporated herein by reference in its entirety for all purposes.

### FIELD OF THE INVENTION

The present invention relates to methods of amplifying samples to preserve epigenetic information and methods for detecting methylation using arrays of nucleic acids.

### BACKGROUND OF THE INVENTION

The genomes of higher eukaryotes contain the modified nucleoside 5-methyl cytosine (5-meC). This modification is usually found as part of the dinucleotide CpG. Cytosine is converted to 5-methylcytosine in a reaction that involves flipping a target cytosine out of an intact double helix and transfer of a methyl group from S-adenosylmethionine by a methyltransferase enzyme (Klimasauskas *et al., Cell* 76:357-369, 1994). This enzymatic conversion is the only epigenetic modification of DNA known to exist in vertebrates and is essential for normal embryonic development (Bird, *Cell* 70:5-8, 1992; Laird and Jaenisch, *Human Mol. Genet.* 3:1487-1495, 1994; and Li *et al., Cell* 69:915-926, 1992).

The frequency of the CpG dinucleotide in the human genome is only about 20% of the statistically expected frequency, possibly because of spontaneous deamination of 5-mCyt to T (Schoreret et al., Proc. Natl. Acad Sci. USA 89:957-961, 1992). Regions where CpG is present at levels that are approximately the expected frequency are referred to as "CpG islands" (Bird, A.P. (1986) *Nature* 321, 209-213). These regions comprise about 1% of vertebrate genomes and account for about 15% of the total number of CpG dinucleotides. CpG islands are typically between 0.2 and 1 kb in length and are located upstream of many housekeeping and tissue-specific genes. CpG islands are often located upstream of transcribed regions, but may also extend into transcribed regions. About 2-4% of cytosines located 5' to guanine are methylated.

DNA methylation is an epigenetic determinant of gene expression. Patterns of CpG methylation are heritable, tissue specific, and correlate with gene expression. DNA methylation also correlates with other cellular processes including chromatin structure, genomic imprinting, somatic X-chromosome inactivation in females and timing of DNA replication. When a gene is highly methylated it is less likely to be expressed, possibly because CpG methylation prevents transcription factors from recognizing their cognate binding sites. Proteins that bind methylated DNA may also recruit histone deacetylase to condense adjacent chromatin. In general transcriptionally inactive genes contain 5-meC whereas transcriptionally active genes do not. Thus the identification of sites in the genome containing 5-meC is important in understanding cell-type specific programs of gene expression and how gene expression profiles are altered during both normal development and diseases such as cancer. Precise mapping of DNA methylation patterns in CpG islands has become essential for understanding diverse biological processes such as the regulation of imprinted genes, X chromosome inactivation, and tumor suppressor gene silencing in human cancer caused by increase methylation.

Methylation of cytosine may lead to decreased gene expression by, for example, disruption of local chromatin structure, inhibition of transcription factor-DNA binding, or by recruitment of proteins which interact specifically with methylated sequences and prevent transcription factor binding. Changes in methylation pattern have been shown to be associated with cancer. Methylation of CpG oligonucleotides in the promoters of tumor suppressor genes may lead to their inactivation. Alterations in the normal methylation process have also been shown to be associated with genomic instability (Lengauer et al. Proc. Natl. Acad. Sci. USA 94:2545-2550, 1997). Such abnormal epigenetic changes may be found in many types of cancer and can serve as potential markers for oncogenic transformation.

All documents, i.e., publications and patent applications, cited in this disclosure, including the foregoing, are incorporated by reference herein in their entireties for all purposes to the same extent as if each of the individual documents were specifically and individually indicated to be so incorporated by reference herein in its entirety.

### SUMMARY OF THE INVENTION

In one aspect of the invention a method for amplifying genomic DNA that copies methylation of cytosines is disclosed. A methylated genomic DNA sample is obtained and amplified by enzymatic extension of primers that are hybridized to the DNA, the primers may be random sequences or locus specific. The primers are hybridized to the methylated DNA and extended using a DNA polymerase activity to generate a hemimethylated hybrid that includes a newly synthesized unmethylated cDNA strand and a methylated template strand. The hemimethylated hybrids may then be treated with a DNA methyltransferase activity in the presence of a methyl donor. The DNA methyltransferase activity methylates hemimethylated sites in the double stranded DNA, to generate methylated hybrids including a newly methylated cDNA strand and a methylated template strand. The methylated hybrids can be denatured and reamplified as described above. The resulting product is an amplified methylated sample. The amplified methylated sample can be analyzed directly to detect cytosines that are methylated and cytosines that are not methylated.

The DNA polymerase may be a thermal stable polymerase or a strand displacing polymerase, such as phi29. In some aspects the DNA methyltranferase activity includes a Dnmt1 enzyme that may be, for example, human or mouse Dnmtl and may be purified from a biological source or may be recombinant or a fusion protein. The Dnmtl activity may be a variant form of human or mouse Dnmtl that has increased specificity for hemimethylated DNA relative to the native enzyme. In preferred aspects, the methyl donor is S-adenosylmethionine.

In one aspect DNA amplified according to the method described above may be treated with methylation specific restriction enzymes to detect methylation of selected cytosines. The amplified methylated DNA may be fragmented with a first restriction enzyme and adaptors may be ligated to the fragments. Aliquots of the adaptor-ligated fragments may be fragmented in parallel with isoschizomers that are differentially sensitive to methylation, for example, one enzyme may be methylation sensitive and the other methylation insensitive or one may be methylation dependent and the other methylation insensitive or methylation sensitive. After fragmentation of the adaptor-ligated fragments the fragments can be amplified with a primer to the adaptor sequence. Fragments that have been cut between the adaptors will not be amplified. The amplified fragments can be labeled and hybridized to an array to detect the presence or absence of different fragments. The hybridization pattern can be analyzed to determine which fragments have been amplified and which have been cut and not amplified efficiently. Amplification can be by any primer mediated extension method, for example, PCR or MDA.

In one aspect a DNA sample is amplified as described above and then cytosine methylation is analyzed by a method that converts unmethylated cytosines in the amplified sample to uracils but does not modify methylated cytosines. The methylation of specific cytosines is then determined by determining the sequence of the products at the position of interest. If it is still a cytosine it was methylated and if it is now a uracil it was unmethylated. Methods for conversion include chemical and enzymatic methods or combinations of the two. Detection can be by hybridization to an array of probes. The probes may be designed to interrogated selected cytosines.

In some aspects antibodies to 5-meC or antibodies to proteins that bind 5-meC selectively are used. Methylated DNA may be enriched in a sample by immunological methods, for example, immunoprecipitation with an antibody to 5-meC or a protein that binds 5-meC and an antibody to that protein.

In one aspect the array of probes includes probes to predicted fragments. The genome of the organism can be analyzed by in silico digestion to predict the size and sequence of restriction fragments and to identify fragments that have CpGs. Probes to fragments of interest can be included on the array. The amplification method may also be taken into account when designing the array. For example, adaptor-mediated PCR amplification generally amplifies fragments of about 200 to 2000 base pairs most efficiently, so fragments in that size range may be targeted by probes. Depending on the enzyme combinations being compared methylation in the fragments can be determined. For example, if a fragment that contains the corresponding restriction site is present in the sample that has been digested with a methylation dependent enzyme that fragment was probably not methylated. HpaII and Mspl is an example of a pair of isoschizomers that are differentially sensitive to methylation and McrBC is an enzyme that is methylation dependent.

In one aspect arrays designed according to the disclosed methods are disclosed. The arrays may have more than 1,000,000, more than 2,000,000 or more than 5,000,000 different probes present at known or determinable locations on a solid support. The array may have probes that are designed to detect fragments that are predicted to be present in a sample after amplification and treatment according to the disclosed methods.

Genomic samples amplified according to the disclosed methods can be analyzed to determine the methylation status of one or more CpG sites present in the sample. Methods that may be used include sodium bisulfite treatment followed by detection of sites of methylation that were not modified by the treatment and remain as C/G base pairs and sites that were not methylated that are modified by the treatment and result in the introduction of an A/T base pair. The presence of the C/G or A/T base pair can be detected by hybridization to an array of probes that are perfectly complementary to the CpG containing region with or without the change introduced by the sodium bisulfite treatment. The A/T or C/G is detected in a manner analogous to the allele specific hybridization detection of a SNP.

In another aspect a method is disclosed for reducing complexity by fragmenting the sample with a methylation dependent endonuclease. The sample is fragmented by a restriction enzyme that cleaves independent of methylation and adaptors are ligated to the fragments. The adaptor-ligated fragments are then digested with the methylation dependent enzyme and a subset of the fragments that are resistant to digestion by the methylation dependent endonuclease are amplified. The amplified fragments can be hybridized to an array to generate a hybridization pattern that is characteristic of the methylation status of the sample. Samples derived from blood, tissue and tumors may be analyzed to generate a hybridization pattern. Hybridization patterns generated this way may be compared to hybridization patterns similarly generated from known samples. Unknown samples can be classified by comparison of hybridization patterns.

### DETAILED DESCRIPTION OF THE INVENTION

### a) General

The present invention has many preferred embodiments and relies on many patents, applications and other references for details known to those of the art. Therefore, when a patent, application, or other reference is cited or repeated below, it should be understood that it is incorporated by reference in its entirety for all purposes as well as for the proposition that is recited.

As used in this application, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as *Genome Analysis: A Laboratory Manual Series (Vols. I-IV)*, *Using Antibodies: A Laboratory Manual, Cells: A Laboratory Manual*, *PCR Primer: A Laboratory Manual,* and Molecular *Cloning: A Laboratory Manual* (all from Cold Spring Harbor Laboratory Press), Stryer, L. (1995) *Biochemistry* (4th Ed.) Freeman, New York, *Gait, "Oligonucleotide Synthesis: A Practical Approach" 1984, IRL Press, London,* Nelson and Cox (2000), *Lehninger, Principles of Biochemistry* 3^{rd} Ed., W.H. Freeman Pub., New York, NY and Berg et al. (2002) *Biochemistry, 5* ^{th} Ed., W.H. Freeman Pub., New York, NY, all of which are herein incorporated in their entirety by reference for all purposes.

The present invention can employ solid substrates, including arrays in some preferred embodiments. Methods and techniques applicable to polymer (including protein) array synthesis have been described in U.S. Serial No. 09/536,841, WO 00/58516, U.S. Patent Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in PCT Applications Nos. PCT/US99/00730 (International Publication No. WO 99/36760) and PCT/USO1/04285 (International Publication No. WO 01/58593), which are all incorporated herein by reference in their entirety for all purposes.

Patents that describe synthesis techniques in specific embodiments include U.S. Patent Nos. 5,412,087, 6,147,205, 6,262,216, 6,310,189, 5,889,165, and 5,959,098. Nucleic acid arrays are described in many of the above patents, but the same techniques are applied to polypeptide arrays.

Nucleic acid arrays that are useful in the present invention include those that are commercially available from Affymetrix (Santa Clara, CA) under the brand name GeneChip®. Example arrays are shown on the website at affymetrix.com.

The present invention also contemplates many uses for polymers attached to solid substrates. These uses include gene expression monitoring, profiling, library screening, genotyping and diagnostics. Gene expression monitoring and profiling methods can be shown in U.S. Patent Nos. 5,800,992, 6,013,449, 6,020,135, 6,033,860, 6,040,138, 6,177,248 and 6,309,822. Genotyping and uses therefore are shown in U.S. Serial Nos. 10/442,021, 10/013,598 (U.S. Patent Application Publication 20030036069), and U.S. Patent Nos. 5,856,092, 6,300,063, 5,858,659, 6,284,460, 6,361,947, 6,368,799 and 6,333,179. Other uses are embodied in U.S. Patent Nos. 5,871,928, 5,902,723, 6,045,996, 5,541,061, and 6,197,506.

The present invention also contemplates sample preparation methods in certain preferred embodiments. Prior to or concurrent with genotyping, the genomic sample may be amplified by a variety of mechanisms, some of which may employ PCR. *See,* for example, *PCR Technology: Principles and Applications for DNA Amplification* (Ed. H.A. Erlich, Freeman Press, NY, NY, 1992); *PCR Protocols: A Guide to Methods and Applications* (Eds. Innis, et al., Academic Press, San Diego, CA, 1990); Mattila et al., *Nucleic Acids Res.* 19, 4967 (1991); Eckert et al., *PCR Methods and Applications* 1, 17 (1991); *PCR* (Eds. McPherson et al., IRL Press, Oxford); and U.S. Patent Nos. 4,683,202, 4,683,195, 4,800,159 4,965,188,and 5,333,675, and each of which is incorporated herein by reference in their entireties for all purposes. The sample may be amplified on the array. See, for example, U.S. Patent No. 6,300,070 and U.S. Serial No. 09/513,300, which are incorporated herein by reference.

Other suitable amplification methods include the ligase chain reaction (LCR) *(for example,* Wu and Wallace, *Genomics* 4, 560 (1989), Landegren et al., *Science* 241, 1077 (1988) and Barringer et al. *Gene* 89:117 (1990)), transcription amplification (Kwoh et at., *Proc. Natl. Acad. Sci. USA* 86, 1173 (1989) and WO88/10315), self-sustained sequence replication (Guatelli et al., *Proc. Nat. Acad. Sci. USA,* 87, 1874 (1990) and WO90/06995), selective amplification of target polynucleotide sequences (U.S. Patent No. 6,410,276), consensus sequence primed polymerase chain reaction (CP-PCR) (U.S. Patent No. 4,437,975), arbitrarily primed polymerase chain reaction (AP-PCR) (U.S. Patent Nos. 5, 413,909, 5,861,245) and nucleic acid based sequence amplification (NABSA). (*See,* U.S. Patent Nos. 5,409,818, 5,554,517, and 6,063,603, each of which is incorporated herein by reference). Other amplification methods that may be used are described in, U.S. Patent Nos. 5,242,794, 5,494,810, 4,988,617 and in U.S. Serial No. 09/854,317, each of which is incorporated herein by reference.

Additional methods of sample preparation and techniques for reducing the complexity of a nucleic sample are described in Dong *et al., Genome Research* 11, 1418 (2001), in U.S. Patent No. 6,361,947, 6,391,592 and 6,107,023 and U.S. Serial Nos. 09/916,135, 09/920,491 (U.S. Patent Application Publication 20030096235), 09/910,292 (U.S. Patent Application Publication 20030082543), and 10/013,598.

Methods for conducting polynucleotide hybridization assays have been well developed in the art. Hybridization assay procedures and conditions will vary depending on the application and are selected in accordance with the general binding methods known including those referred to in: Maniatis *et al. Molecular Cloning: A Laboratory Manual* (2°^{d} Ed. Cold Spring Harbor, N.Y, 1989); Berger and Kimmel *Methods in Enzymology,* Vol. 152, *Guide to Molecular Cloning* *Techniques* (Academic Press, Inc., San Diego, CA, 1987); Young and Davism, *P.N.A.S,* 80: 1194 (1983). Methods and apparatus for carrying out repeated and controlled hybridization reactions have been described in U.S. Patent Nos. 5,871,928, 5,874,219, 6,045,996 and 6,386,749, 6,391,623 each of which are incorporated herein by reference.

The present invention also contemplates signal detection of hybridization between ligands in certain preferred embodiments. See U.S. Patent Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Serial No. 10/389,194 and in PCT Application PCT/US99/06097 (published as WO99/47964), each of which also is hereby incorporated by reference in its entirety for all purposes.

Methods and apparatus for signal detection and processing of intensity data are disclosed in, for example, U.S. Patents Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Serial Nos. 10/389,194, 60/493,495 and in PCT Application PCT/US99/06097 (published as WO 99/47964), each of which also is hereby incorporated by reference in its entirety for all purposes. Instruments and software may also be purchased commercially from various sources, including Affymetrix.

The practice of the present invention may also employ conventional biology methods, software and systems. Computer software products of the invention typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, for example Setubal and Meidanis et al., *Introduction to Computational Biology Methods* (PWS Publishing Company, Boston, 1997); Salzberg, Searles, Kasif, (Ed.), *Computational Methods in Molecular Biology,* (Elsevier, Amsterdam, 1998); Rashidi and Buehler, *Bioinformatics Basics: Application in Biological Science and Medicine* (CRC Press, London, 2000) and Ouelette and Bzevanis *Bioinformatics: A Practical Guide for Analysis of Gene and Proteins* (Wiley & Sons, Inc., 2^{nd} ed., 2001). See U.S. Patent No. 6,420,108.

The present invention may also make use of various computer program products and software for a variety of purposes, such as probe design, management of data, analysis, and instrument operation. See, U.S. Patent Nos. 5,593,839, 5,795,716, 5,733,729, 5,974,164, 6,066,454, 6,090,555, 6,185,561, 6,188,783, 6,223,127, 6,229,911 and 6,308,170.

Additionally, the present invention may have preferred embodiments that include methods for providing genetic information over networks such as the Internet as shown in U.S. Serial Nos. 10/197,621, 10/063,559 (United States Publication No. 20020183936), 10/065,856, 10/065,868, 10/328,818, 10/328,872, 10/423,403, and 60/482,389.

### b) Definitions

"Adaptor sequences" or "adaptors" are generally oligonucleotides of at least 5, 10, or 15 5 bases and preferably no more than 50 or 60 bases in length; however, they may be even longer, up to 100 or 200 bases. Adaptor sequences may be synthesized using any methods known to those of skill in the art. For the purposes of this invention they may, as options, comprise primer binding sites, recognition sites for endonucleases, common sequences and promoters. The adaptor may be entirely or substantially double stranded or entirely single stranded. A double stranded adaptor may comprise two oligonucleotides that are at least partially complementary. The adaptor may be phosphorylated or unphosphorylated on one or both strands.

Adaptors may be more efficiently ligated to fragments if they comprise a substantially double stranded region and a short single stranded region which is complementary to the single stranded region created by digestion with a restriction enzyme. For example, when DNA is digested with the restriction enzyme *Eco*RI the resulting double stranded fragments are flanked at either end by the single stranded overhang 5'-AATT-3', an adaptor that carries a single stranded overhang 5'-AATT-3' will hybridize to the fragment through complementarity between the overhanging regions. This "sticky end" hybridization of the adaptor to the fragment may facilitate ligation of the adaptor to the fragment but blunt ended ligation is also possible. Blunt ends can be converted to sticky ends using the exonuclease activity of the Klenow fragment. For example when DNA is digested with PvuII the blunt ends can be converted to a two base pair overhang by incubating the fragments with Klenow in the presence of dTTP and dCTP. Overhangs may also be converted to blunt ends by filling in an overhang or removing an overhang.

Methods of ligation will be known to those of skill in the art and are described, for example in Sambrook et at. (2001) and the New England BioLabs catalog both of which are incorporated herein by reference for all purposes. Methods include using T4 DNA Ligase which catalyzes the formation of phosphodiester bond between juxtaposed 5' phosphate and 3' hydroxyl termini in duplex DNA or RNA with blunt and sticky ends; *Taq* DNA Ligase which catalyzes the formation of a phosphodiester bond between juxtaposed 5' phosphate and 3' hydroxyl termini of two adjacent oligonucleotides which are hybridized to a complementary target DNA; *E. coli* DNA ligase which catalyzes the formation of a phosphodiester bond between juxtaposed 5' -phosphate and 3' -hydroxyl termini in duplex DNA containing cohesive ends; and T4 RNA ligase which catalyzes ligation of a 5' phosphoryl-terminated nucleic acid donor to a 3' hydroxyl-terminated nucleic acid acceptor through the formation of a 3' ->5' phosphodiester bond, substrates include single-stranded RNA and DNA as well as dinucleoside pyrophosphates; or any other methods described in the art. Fragmented DNA may be treated with one or more enzymes, for example, an endonuclease, prior to ligation of adaptors to one or both ends to facilitate ligation by generating ends that are compatible with ligation.

Adaptors may also incorporate modified nucleotides that modify the properties of the adaptor sequence. For example, phosphorothioate groups may be incorporated in one of the adaptor strands. A phosphorothioate group is a modified phosphate group with one of the oxygen atoms replaced by a sulfur atom. In a phosphorothioated oligo (often called an "S-Oligo"), some or all of the intemucleotide phosphate groups are replaced by phosphorothioate groups. The modified backbone of an S-Oligo is resistant to the action of most exonucleases and endonucleases. Phosphorothioates may be incorporated between all residues of an adaptor strand, or at specified locations within a sequence. A useful option is to sulfurize only the last few residues at each end of the oligo. This results in an oligo that is resistant to exonucleases, but has a natural DNA center.

The term "array" as used herein refers to an intentionally created collection of molecules which can be prepared either synthetically or biosynthetically. The molecules in the array can be identical or different from each other. The array can assume a variety of formats, *for example,* libraries of soluble molecules; libraries of compounds tethered to resin beads, silica chips, or other solid supports.

The term "array plate" as used herein refers to a body having a plurality of arrays in which each microarray is separated by a physical barrier resistant to the passage of liquids and forming an area or space, referred to as a well, capable of containing liquids in contact with the probe array.

The term "biomonomer" as used herein refers to a single unit of biopolymer, which can be linked with the same or other biomonomers to form a biopolymer (for example, a single amino acid or nucleotide with two linking groups one or both of which may have removable protecting groups) or a single unit which is not part of a biopolymer. Thus, for example, a nucleotide is a biomonomer within an oligonucleotide biopolymer, and an amino acid is a biomonomer within a protein or peptide biopolymer; avidin, biotin, antibodies, antibody fragments, etc., for example, are also biomonomers.

The term "biopolymer" or sometimes refer by "biological polymer" as used herein is intended to mean repeating units of biological or chemical moieties. Representative biopolymers include, but are not limited to, nucleic acids, oligonucleotides, amino acids, proteins, peptides, hormones, oligosaccharides, lipids, glycolipids, lipopolysaccharides, phospholipids, synthetic analogues of the foregoing, including, but not limited to, inverted nucleotides, peptide nucleic acids, Meta-DNA, and combinations of the above.

The term "combinatorial synthesis strategy" as used herein refers to a combinatorial synthesis strategy is an ordered strategy for parallel synthesis of diverse polymer sequences by sequential addition of reagents which may be represented by a reactant matrix and a switch matrix, the product of which is a product matrix. A reactant matrix is a I column by m row matrix of the building blocks to be added. The switch matrix is all or a subset of the binary numbers, preferably ordered, between 1 and m arranged in columns. A "binary strategy" is one in which at least two successive steps illuminate a portion, often half, of a region of interest on the substrate. In a binary synthesis strategy, all possible compounds which can be formed from an ordered set of reactants are formed. In most preferred embodiments, binary synthesis refers to a synthesis strategy which also factors a previous addition step. For example, a strategy in which a switch matrix for a masking strategy halves regions that were previously illuminated, illuminating about half of the previously illuminated region and protecting the remaining half (while also protecting about half of previously protected regions and illuminating about half of previously protected regions). It will be recognized that binary rounds may be interspersed with non-binary rounds and that only a portion of a substrate may be subjected to a binary scheme. A combinatorial "masking" strategy is a synthesis which uses light or other spatially selective deprotecting or activating agents to remove protecting groups from materials for addition of other materials such as amino acids.

The term "complementary" as used herein refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, M. Kanehisa Nucleic Acids Res. 12:203 (1984), incorporated herein by reference.

The term "epigenetic" as used herein refers to factors other than the primary sequence of the genome that affect the development or function of an organism, they can affect the phenotype of an organism without changing the genotype. Epigenetic factors include modifications in gene expression that are controlled by heritable but potentially reversible changes in DNA methylation and chromatin structure. Methylation patterns are known to correlate with gene expression and in general highly methylated sequences are poorly expressed.

The term "genome" as used herein is all the genetic material in the chromosomes of an organism. DNA derived from the genetic material in the chromosomes of a particular organism is genomic DNA. A genomic library is a collection of clones made from a set of randomly generated overlapping DNA fragments representing the entire genome of an organism.

The term "hybridization" as used herein refers to the process in which two single-stranded polynucleotides bind non-covalently to form a stable double-stranded polynucleotide; triple-stranded hybridization is also theoretically possible. The resulting (usually) double-stranded polynucleotide is a "hybrid." Hybridizations are usually performed under stringent conditions, for example, at a salt concentration of no more than about 1 M and a temperature of at least 25°C. For example, conditions of 5X SSPE (750 mM NaCI, 50 mM NaPhosphate, 5 mM EDTA, pH 7.4) and a temperature of 25-30°C are suitable for allele-specific probe hybridizations or conditions of 100 mM MES, 1 M [Na⁺], 20 mM EDTA, 0.01% Tween-20 and a temperature of 30-50°C, preferably at about 45-50°C. Hybridizations may be performed in the presence of agents such as herring sperm DNA at about 0.1 mg/ml, acetylated BSA at about 0.5 mg/ml. As other factors may affect the stringency of hybridization, including base composition and length of the complementary strands, presence of organic solvents and extent of base mismatching, the combination of parameters is more important than the absolute measure of any one alone. Hybridization conditions suitable for microarrays are described in the Gene Expression Technical Manual, 2004 and the GeneChip Mapping Assay Manual, 2004, available at Affymetrix.com.

The term "hybridization probes" as used herein are oligonucleotides capable of binding in a base-specific manner to a complementary strand of nucleic acid. Such probes include peptide nucleic acids, as described in Nielsen et al., *Science* 254, 1497-1500 (1991), LNAs, as described in Koshkin et al. *Tetrahedron* 54:3607-3630, 1998, and US Patent No. 6,268,490 and other nucleic acid analogs and nucleic acid mimetics.

The term "isolated nucleic acid" as used herein mean an object species invention that is the predominant species present (*i.e*., on a molar basis it is more abundant than any other individual species in the composition). Preferably, an isolated nucleic acid comprises at least about 50, 80 or 90% (on a molar basis) of all macromolecular species present. Most preferably, the object species is purified to essential homogeneity (contaminant species cannot be detected in the composition by conventional detection methods).

The term "label" as used herein refers to a luminescent label, a light scattering label or a radioactive label. Fluorescent labels include, *inter alia,* the commercially available fluorescein phosphoramidites such as Fluoreprime (Pharmacia), Fluoredite (Millipore) and FAM (ABI). See United States Patent 6,287,778.

The term "ligand" as used herein refers to a molecule that is recognized by a particular receptor. The agent bound by or reacting with a receptor is called a "ligand," a term which is definitionally meaningful only in terms of its counterpart receptor. The term "ligand" does not imply any particular molecular size or other structural or compositional feature other than that the substance in question is capable of binding or otherwise interacting with the receptor. Also, a ligand may serve either as the natural ligand to which the receptor binds, or as a functional analogue that may act as an agonist or antagonist. Examples of ligands that can be investigated by this invention include, but are not restricted to, agonists and antagonists for cell membrane receptors, toxins and venoms, viral epitopes, hormones (for example, opiates, steroids, etc.), hormone receptors, peptides, enzymes, enzyme substrates, substrate analogs, transition state analogs, cofactors, drugs, proteins, and antibodies.

The term "mixed population" or sometimes refer by "complex population" as used herein refers to any sample containing both desired and undesired nucleic acids. As a non-limiting example, a complex population of nucleic acids may be total genomic DNA, total genomic RNA or a combination thereof. Moreover, a complex population of nucleic acids may have been enriched for a given population but include other undesirable populations. For example, a complex population of nucleic acids may be a sample which has been enriched for desired messenger RNA (mRNA) sequences but still includes some undesired ribosomal RNA sequences (rRNA).

The term "mRNA" or sometimes refer by "mRNA transcripts" as used herein, include, but not limited to pre-mRNA transcript(s), transcript processing intermediates, mature mRNA(s) ready for translation and transcripts of the gene or genes, or nucleic acids derived from the mRNA transcript(s). Transcript processing may include splicing, editing and degradation. As used herein, a nucleic acid derived from an mRNA transcript refers to a nucleic acid for whose synthesis the mRNA transcript or a subsequence thereof has ultimately served as a template. Thus, a cDNA reverse transcribed from an mRNA, an RNA transcribed from that cDNA, a DNA amplified from the cDNA, an RNA transcribed from the amplified DNA, *etc*., are all derived from the mRNA transcript and detection of such derived products is indicative of the presence and/or abundance of the original transcript in a sample. Thus, mRNA derived samples include, but are not limited to, mRNA transcripts of the gene or genes, cDNA reverse transcribed from the mRNA, cRNA transcribed from the cDNA, DNA amplified from the genes, RNA transcribed from amplified DNA, and the like.

The term "nucleic acid library" as used herein refers to an intentionally created collection of nucleic acids which can be prepared either synthetically or biosynthetically and screened for biological activity in a variety of different formats (for example, libraries of soluble molecules; and libraries of oligos tethered to beads, chips, or other solid supports). Additionally, the term "array" is meant to include those libraries of nucleic acids which can be prepared by spotting nucleic acids of essentially any length (for example, from 1 to about 1000 nucleotide monomers in length) onto a substrate. The term "nucleic acid" as used herein refers to a polymeric form of nucleotides of any length, either ribonucleotides, deoxyribonucleotides or peptide nucleic acids (PNAs), that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into a nucleic acid or oligonucleoside sequence, they allow hybridization with a naturally occurring nucleic acid sequence in solution. Typically, these analogs are derived from naturally occurring nucleosides and nucleotides by replacing and/or modifying the base, the ribose or the phosphodiester moiety. The changes can be tailor made to stabilize or destabilize hybrid formation or enhance the specificity of hybridization with a complementary nucleic acid sequence as desired.

The term "nucleic acids" as used herein may include any polymer or oligomer of pyrimidine and purine bases, preferably cytosine, thymine, and uracil, and adenine and guanine, respectively. See Albert L. Lehninger, PRINCIPLES OF BIOCHEMISTRY, at 793-800 (Worth Pub. 1982). Indeed, the present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "oligonucleotide" or sometimes refer by "polynucleotide" as used herein refers to a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA) which may be isolated from natural sources, recombinantly produced or artificially synthesized and mimetics thereof. A further example of a polynucleotide of the present invention may be peptide nucleic acid (PNA). The invention also encompasses situations in which there is a nontraditional base pairing such as Hoogsteen base pairing which has been identified in certain tRNA molecules and postulated to exist in a triple helix. "Polynucleotide" and "oligonucleotide" are used interchangeably in this application.

The term "primer" as used herein refers to a single-stranded oligonucleotide capable of acting as a point of initiation for template-directed DNA synthesis under suitable conditions for example, buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. Short primer molecules generally require cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

The term "probe" as used herein refers to a surface-immobilized molecule that can be recognized by a particular target. See U.S. Patent No. 6,582,908 for an example of arrays having all possible combinations of probes with 10, 12, and more bases. Examples of probes that can be investigated by this invention include, but are not restricted to, agonists and antagonists for cell membrane receptors, toxins and venoms, viral epitopes, hormones (for example, opioid peptides, steroids, etc.), hormone receptors, peptides, enzymes, enzyme substrates, cofactors, drugs, lectins, sugars, oligonucleotides, nucleic acids, oligosaccharides, proteins, and monoclonal antibodies.

The term "receptor" as used herein refers to a molecule that has an affinity for a given ligand. Receptors may be naturally-occurring or manmade molecules. Also, they can be employed in their unaltered state or as aggregates with other species. Receptors may be attached, covalently or noncovalently, to a binding member, either directly or via a specific binding substance. Examples of receptors which can be employed by this invention include, but are not restricted to, antibodies, cell membrane receptors, monoclonal antibodies and antisera reactive with specific antigenic determinants (such as on viruses, cells or other materials), drugs, polynucleotides, nucleic acids, peptides, cofactors, lectins, sugars, polysaccharides, cells, cellular membranes, and organelles. Receptors are sometimes referred to in the art as anti-ligands. As the term receptors is used herein, no difference in meaning is intended. A "Ligand Receptor Pair" is formed when two macromolecules have combined through molecular recognition to form a complex. Other examples of receptors which can be investigated by this invention include but are not restricted to those molecules shown in U.S. Patent No. 5,143,854, which is hereby incorporated by reference in its entirety.

The term "solid support", "support", and "substrate" as used herein are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. See U.S. Patent No. 5,744,305 for exemplary substrates.

The term "target" as used herein refers to a molecule that has an affinity for a given probe. Targets may be naturally-occurring or man-made molecules. Also, they can be employed in their unaltered state or as aggregates with other species. Targets may be attached, covalently or noncovalently, to a binding member, either directly or via a specific binding substance. Examples of targets which can be employed by this invention include, but are not restricted to, antibodies, cell membrane receptors, monoclonal antibodies and antisera reactive with specific antigenic determinants (such as on viruses, cells or other materials), drugs, oligonucleotides, nucleic acids, peptides, cofactors, lectins, sugars, polysaccharides, cells, cellular membranes, and organelles. Targets are sometimes referred to in the art as anti-probes. As the term targets is used herein, no difference in meaning is intended. A "Probe Target Pair" is formed when two macromolecules have combined through molecular recognition to form a complex.

The term "wafer" as used herein refers to a substrate having surface to which a plurality of arrays are bound. In a preferred embodiment, the arrays are synthesized on the surface of the substrate to create multiple arrays that are physically separate. In one preferred embodiment of a wafer, the arrays are physically separated by a distance of at least about 0.1, 0.25, 0.5, 1 or 1.5 millimeters. The arrays that are on the wafer may be identical, each one may be different, or there may be some combination thereof. Particularly preferred wafers are about 8" x 8" and are made using the photolithographic process.

An individual is not limited to a human being, but may also include other organisms including but not limited to mammals, plants, fungi, bacteria or cells derived from any of the above.

### Methylation Analysis

Methylation of cytosine residues in DNA plays an important role in gene regulation. DNA methylation is required for normal embryonic development and changes in methylation are often associated with disease. Genomic imprinting, X chromosome inactivation, chromatin modification, and silencing of endogenous retroviruses all depend on establishing and maintaining proper methylation patterns. Expression of genes may be regulated by patterns of methylation. Abnormal methylation is a hallmark of cancer cells and silencing of tumor suppressor genes is thought to be a causal basis for many human cancers. Methylation mapping using microarray-based approaches may be used, for example, to profile cancer cells revealing a pattern of DNA methylation that may be used, for example, to diagnose a malignancy, predict treatment outcome or monitor progression of disease. Methylation in eukaryotes can also function to inhibit the activity of viruses and transposons, see Jones *et al., EMBO J.* 17:6385-6393 (1998).

In preferred aspects, methods are disclosed for analysis of methylation status of cytosines in genomic samples using hybridization to nucleic acid probe arrays. In many aspects the methods include a step that distinguishes between methylated and unmethylated cytosines. The step may be, for example, chemical or enzymatic. For example, in some aspects a sample containing methylated cytosines is treated with bisulfite, which selectively modifies unmethylated cytosines. In other aspects, enzymes that differentiate between methylated and unmethylated DNA are used, for example, DNA may be digested in parallel with isoschizomers where one enzyme is methylation sensitive and the other is methylation insensitive.

In some aspects methods for amplifying genomic DNA samples while preserving information about the methylation status of cytosines in the DNA are disclosed. The amplified sample may then be analyzed for methylation status of one or more cytosines. This method of amplification with preservation of methylation status may be particularly useful when combined with a detection method such as bisulfite modification because bisulfite modification and other similar treatments may damage DNA and methods to amplify the DNA post modification may be inefficient as a result. Amplification of the sample before modification or treatment may allow detection of the modified sample directly without the need to amplify the sample post modification or treatment. Methods are further disclosed for detecting the methylation status of cytosines. In preferred embodiments the methods are capable of simultaneously analyzing large numbers of cytosines, for example, more than 1,000, 5,000, 10,000, or 100,000 cytosines.

Many methods of analysis of genomic information employ an amplification step prior to analysis and typically these methods result in loss of information related to methylation status of the starting material because the sequence of the starting material is reproduced but epigenetic information, such as the presence of methylation is lost. In one aspect of the present invention methods for amplification of nucleic acids with retention of at least some epigenetic information are disclosed.

Amplification may be by a variety of means, such as polymerase chain reaction (PCR), rolling circle amplification (RCA), multiple displacement amplification (MDA), or other methods of whole genome amplification, representative genome amplification or locus specific amplification. Methods may include fragmenting the nucleic acid sample, for example, with one or more restriction endonucleases or by a random fragmentation method, and attaching a universal priming site by, for example, ligation of an adaptor or extension of primers with a universal 5' end and random or degenerate sequence at the 3' end.

For many amplification methods a primer is hybridized to the genomic template and is extended creating a cDNA strand that is a copy of the genomic DNA. First strand cDNA synthesis results in a hybrid between the *in vitro* synthesized cDNA and the *in vivo* synthesized genomic DNA strand, but typically the newly synthesized cDNA is not methylated because the system responsible for maintaining methylation status *in vivo* is absent from the in vitro reaction. In one embodiment of the present invention, amplification of genomic DNA is done in the presence of an activity that is capable of preserving methylation by identifying methylation on the parent, template strand and methylating the newly synthesized, daughter strand at the corresponding site.

In a preferred embodiment the first strand cDNA is methylated while in a complex with the genomic DNA strand used as a template to generate the cDNA. The now methylated first strand cDNA (daughter) and the template genomic DNA strand (parent) are then separated and may be used as templates for synthesis of additional strands, the methylated daughter serving as template and parent for a subsequent newly synthesized daughter strand. Each successive strand generated is methylated using its template (parent) strand as a guide for methylation.

Mammalian methylation patterns are complex and change during development, see van Steensel and Henikoff *BioTechniques* 35: 346-357 (2003). Methylation typically occurs at the 5 position of cytosine to generate 5-methylcytosine (5-meC). Most methyl cytosines are found at CpG dinucleotides. Methylation in promoter regions is generally accompanied by gene silencing and loss of methylation or loss of the proteins that bind to the methylated CpG can lead to diseases in humans, for example, Immunodeficiency Craniofacial Syndrome and Rett Syndrome, Bestor (2000) *Hum. Mol. Genet.* 9:2395-2402.

Processes have evolved to maintain methylation through multiple rounds of cell division and even through germ lines, see Raykan and Whitelaw (2003) *Curr. Biol.* 13:R6. In animals methyl-CpG is maintained by DNA methyltransferase 1 (Dnmt 1). Dnmt 1 follows behind the replication fork and methylates the unmethylated C residue of each CpG that is base paired with methyl-CpG, see Leonhardt et al. (1992) Cell 71:865-873. Dnmt 1 is specific for hemimethylated sites thus preserving methylation sites through successive rounds of cell division. DNA methylation is gene-specific and occurs genome-wide.

Two distinct processes modulate methylation, maintenance methylation after DNA replication and de novo application of methyl groups in previously unmethylated regions. In mammals, one class of methyltransferases is responsible for methylating unmodified DNA and is designated as the de novo enzyme. Another class maintains the methylation status of the daughter strand during DNA replication and is referred to as a maintenance DNA methyltransferase. Both classes of enzyme catalyze the transfer of a methyl group from S-adenosyl-L-methionine (AdoMet) to cytosine bases in DNA. Maintenance methylation is responsible for adding methyl groups to cytosines in newly synthesized DNA after replication and cell division. The methylated sites in the parental DNA serve as template for correct methylation by maintenance methyltransferase activities that recognize hemimethylated daughter strands soon after replication, see Riggs, A.D. (1989) *Cell Biophys.* 15:1-13. In contrast, de novo methylation activities create new methylation patterns. DNA methyltransferases (DNMTs) are found in many organisms including mammals, plants and bacteria (Bestor et al. *Curr. Opin. Cell Biol.,* 6:380-389 (1994). In mammals, three active DNA methyltransferases have been identified, Dnmtl, Dnmt3a and Dnmt3b. Dnmt2 has also been recently identified. Dnmt3a and 3b function primarily as de novo methyltransferases while Dnmt 1 is responsible for maintenance methylation. Dnmtl has been shown to have a high preference for methylation of hemimethylated CpG sites in vitro, see Pradhan et al. (1997) *Nucl. Acids Res.* 25:4666-4673 and (1999) *J. Biol. Chem.* 274:33002-33010.

Dnmtl, Dnmt3a and Dnmt3b are active in vitro in the presence of substrate DNA and AdoMet cofactor. Dnmt is the most abundant DNA methyltransferases in mammalian cells and has been purified from native sources. Recombinant Dnmtl is also available from several organisms, including mouse and human. The enzyme is 7 to 20 fold more active on hemimethylated DNA as compared to unmethylated substrate in vivo. Dnmtl has been shown to have several naturally occurring isoforms including a splice variant form, Dnmt1b and an oocyte specific form, Dnmtlo. Mutational analysis of the Dnmt1 protein has identified a carboxy-terminal catalytic domain and a regulatory domain containing a nuclear localization signal, replication fork targeting peptide and zinc binding region. A number of specific protein interaction regions have also been identified in the protein. For a review see, Pradhan and Esteve, *Clinical Immunology* 109:6-16 (2003).

Human DNA (cytosine-5) methyltranferase (Dnmt1) is commercially available from New England Biolabs. The protein is expressed from a human cDNA using a baculovirus expression system. The enzyme is provided with a 10x reaction buffer, BSA and S-adenosylmethionine (SAM). The recommended reaction conditions are 1X Dnmtl reaction buffer (IX is 59 mM Tris-HCI, 1 mM DTT, 1 mM EDTA, 5% glycerol, pH7.8 at 25°C), 100 µg/ml BSA and 160 µM SAM at 37°C. 1 unit of enzyme is defined as the amount of enzyme required to catalyze the transfer of 1 pmol of methyl group to poly dI.dC substrate in a total reaction volume of 25 µl in 30 minutes at 37°C. An antibody to the enzyme is also available.

A recent study of Dnmt3a showed that the catalytic activity of the enzyme is stimulated in vitro by DMSO, see Yokochi and Robertson, Bioorganic Chem. 32:234-243 (2004). This study suggests that the DMSP stimulation effect depends on an interaction between DMSO and the enzymes reaction substrates, DNA and AdoMet, and not the enzyme itself. In some aspects DMSO may be included in a Dnmtl reaction.

In one aspect of the invention a sample of genomic DNA containing methylated cytosines is obtained and one or more primers are hybridized to the DNA. The primers may be, for example, one or more locus specific primers or a collection or random or partially degenerate primers. The primers are extended with a DNA polymerase to form double stranded DNA hybrids. The hybrids contain one strand of template DNA and one strand of newly synthesized cDNA. The genomic template strand may contain sites of methylation, while the newly synthesized strand may be unmethylated. The hybrids are treated with a DNA methyltransferase activity that recognizes sites of methylation in the template strand and methylates the newly synthesized strand at the sites of methylation in the template strand. The methyltransferase and DNA polymerase activities may function simultaneously or at different times. After methylation of the newly synthesized strand is complete the newly synthesized, methylated strand may be used as a template strand in a second round of amplification and methylation. The steps may be repeated multiple times to generate many copies of the starting genomic template, including sites of methylation. In preferred embodiments, copying of the methylation of the template is allowed to go to completion prior to a subsequent round of amplification. In some embodiments the reaction may be treated with an enzyme that cleaves hemimethylated sites prior to a subsequent round of amplification.

In one embodiment, PCR is used for amplification and the DNA methyltransferase activity is thermostable and will retain activity during successive rounds of heating to denature the double stranded DNA. In another embodiment, amplification is done using a strand displacing enzyme such as phi29 (see, for example, US Patent Nos. 6,617,137 and 6,323,009) and the DNA methyltransferase may or may not be thermostable. When strand displacing polymerases are employed the methyltransferases activity preferably acts in conjunction with the polymerase so that methylation sites are copied into the daughter strand before the daughter strand is displaced by a subsequent daughter strand. In one aspect the amplification is isothermal.

Following amplification, the amplified material with methylation status preserved may be assayed to determine the methylation status of one or more cytosines. Any available method for determining methylation status may be used. For methods of detecting methylation status see, for example U.S. Patent Application No. 10/841,027, US Patent Nos. 6,214,556, 5,786,146, 6,017,704, 6,265,171, 6,200,756, 6,251,594, 5,912,147, 6,331,393, 6,605,432, and 6,300,071 and US Patent Application publications 20030148327, 20030148326, 20030143606 and 20030082609. For a review of some methylation detection methods, see, Oakeley, E.J., *Pharmacology & Therapeutics* 84:389-400 (1999). Available methods include: reverse-phase HPLC, thin-layer chromatography, SssI methyltransferases with incorporation of labeled methyl groups, the chloracetaldehyde reaction, differentially sensitive restriction enzymes, hydrazine or permanganate treatment (m5C is cleaved by permanganate treatment but not by hydrazine treatment), sodium bisulfite, combined bisulphate-restriction analysis, and methylation sensitive single nucleotide primer extension. Each of these is described in Oakeley (1999). For many of these methods, preservation of methylation status during amplification facilitates downstream methods of analysis that rely on treatments that are sensitive to methylation. Many methods of detecting methylation employ an amplification step after a methylation sensitive modification step, in some aspects the need to amplify the sample after modification is eliminated by amplification with preservation of methylation status before treatment. This may improve the efficiency of amplification and detection. Post modification amplification can be inefficient as a result of damage to the DNA during the modification step.

In one aspect of the invention the methylation status of a cytosine is analyzed using restriction digestion with two restriction enzymes that recognize the same recognition site but are differentially sensitive to methylation. An example of such an enzyme pair is HpaII and Mspl. HpaII and MspI are isoschizomers that cleave at the recognition site CCGG (see, New England Biolab Catalogue, which is incorporated herein by reference in its entirety). Cleavage by HpaII is blocked by methylation of the central C while Mspl cleaves independent of methylation of the central C. In one aspect of the invention an amplified sample with preserved methylation information is subjected to digestion with Mspl and HpaII and cleavage products may be analyzed to determine methylation status. If the site of interest is methylated it will not be cleaved by HpaII but will be cleaved by Mspl.

In an exemplary embodiment, the sample is first fragmented with a restriction enzyme such as XbaI, HindIII or BglII and adaptors are ligated to the fragments. In preferred embodiments the first restriction enzyme does not contain CpG in its recognition site. Multiple enzymes may be used for the first fragmentation. After adaptor ligation the sample is divided into fractions or aliquots that are then subjected to different treatments and may be analyzed by, for example, parallel hybridization to an array of probes. In one aspect one fraction is fragmented with Hpa II and a second fraction is fragmented with Mspl. A third fraction may also be included that is not fragmented with either MspI or HpaII. The HpaII digested fraction may be compared to the MspI digested fraction or to the undigested fraction or to both. HpaII and Mspl are used herein as exemplary enzymes, but any pair of enzymes that recognize the same restriction site and are differentially sensitive to methylation may be used.

Fragments that have the CCGG recognition site will either be cleaved in both the Mspl and HpaII fractions if the CpG is unmethylated or will be cleaved in the MspI fraction but not in the HpaII fraction if the CpG is methylated. After cleavage the samples are amplified using one or more primers that are complementary to the adaptor. It is not necessary to maintain information about methylation status during this amplification step. Adaptor-ligated fragments that have been digested with Mspl or with HpaII, because the site was not methylated, will not amplify during the PCR reaction. If a fragment has been cleaved by MspI or HpaII the fragment will not be amplified in the PCR reaction because the resulting fragments will have the adaptor sequence, and therefore the priming site, only on one end. Fragments that have only methylated CCGG sites will not be cleaved in the HpaII reaction and they will be amplified during PCR. The fragments that are present in the HpaII reaction but not in the Mspl reaction can be identified by hybridizing the products of each of the PCR amplification reaction to an array of probes. Probes that detect hybridization above background only in the HpaII reaction are indicative of a methylated fragment.

In another aspect, an enzyme that cleaves only methylated but not unmethylated DNA may be used. Adaptor-ligated fragments, generated as described above, are digested with a methyl-dependent enzyme that cleaves only methylated DNA and the undigested adaptor-ligated fragments are amplified with a primer to the adaptor sequence. Adaptor-ligated fragments that have been digested with the methyl-dependent enzyme are not amplified and are not detected in subsequent detection steps. The products of the amplification are hybridized to an array and a hybridization pattern is obtained and compared to a hybridization pattern resulting from a sample treated in parallel but not digested with the methyl-dependent enzyme. Differences between the two patterns are indicative of fragments that were methylated in the sample. Methyl-dependent enzymes include, for example, McrBC.

The amplification products may be detected by any method known in the art, for example by hybridization to an array of probes. The array may have probes to selected regions or probes tiled to represent an entire chromosome, an entire genome, or one or more large regions of a genome. The array may also be designed with probes to regions containing predicted or known methylation sites. Exemplary arrays include the arrays disclosed in US patent application nos. 10/264,945, 09/916,135 and 60/417,190 each of which is incorporated herein by reference.

In one aspect a computer is used to model the products of the first restriction enzyme digestion to predict the size and sequence of fragments. A computer may then be used to identify those fragments that also contain one or more recognition sites for a methylation sensitive restriction enzyme. A computer may also be used to identify fragments that are amenable to amplification by the PCR conditions. In many embodiments the PCR conditions preferentially amplify fragments of a limited size range, for example, 200 to 2,000 base pairs or 200 to 1,000 base pairs. Fragments that are within the expected size range that also contain a site for a methylation sensitive enzyme are identified and an array may be designed with probes complementary to a plurality of the fragments that are identified.

In an exemplary aspect the first restriction enzyme is Xbal, and a computer is used to model digestion of the human genome by XbaI to identify Xbal fragments that are between 200 and 1,000 base pairs. A computer is used to analyze the sequence of the identified fragments to identify a subset of fragments that have at least one CCGG site within the fragment. Probes of the array may be designed to interrogate those fragments that meet both criteria. The probes may be to any region of the fragment and preferably each fragment is interrogated by a plurality of different sequence probes that are perfectly complementary to different, but optionally overlapping, regions of the fragment. In one aspect, the hybridization pattern resulting from the HpaII digested fragments is compared to a Mspl digested reaction. Fragments that contain a CCGG site but are amplified after HpaII digestion are indicative of methylation of the CCGG sites in that fragment. The fragment should not be detected in the Mspl digested reaction. In another aspect the HpaII hybridization pattern is compared to the hybridization pattern of a sample that is not treated with the second digestion. Without the second digestion both methylated and unmethylated fragments will be amplified and detected by hybridization to the array, serving as a positive control for the amplification. Fragments carrying only unmethylated CCGG sequences will be digested in the HpaII reaction so they will not be amplified in the subsequent amplification step and the probes to those fragments should not generate signal above background. The sample that is not treated with a second digestion step can also be used to estimate the level of methylation. If the fragment is only partially methylated and there is a mixture of methylated and unmethylated for the fragment, the intensity of the signal may be compared to the intensity of signal from the untreated sample to estimate the amount of the fragment that is methylated.

In one aspect, a computer system is used to locate and map methylated fragments in the genome based on the expected products of the first fragmentation reaction and the sequence of the probe showing hybridization. In addition a computer may be used to identify CCGG sites in the identified fragment.

In one aspect of the invention, the array of probes comprises probes that are complementary to regions of the genome that contain CpG islands. The probes may be designed to be complementary to a region that will be in the same restriction fragment as the CpG island, but may be complementary to a region that does not contain CpG dinucleotides.

In one embodiment, methylation status in the amplified, methylated product may be analyzed by the sodium bisulfite treatment method. Unmethylated cytosine is converted to uracil through a three-step process during sodium bisulfite modification. The steps are sulphonation to convert cytosine to cytosine sulphonate, deamination to convert cytosine sulphonate to uracil sulphonate and alkali desulphonation to convert uracil sulphonate to uracil. Conversion does not occur on methylated cytosine. *See* Clark et al. *Nucleic Acids Res.,* 22(15):2990-7 (1994). If the cytosine is methylated it will remain a cytosine. If the cytosine is unmethylated it will be converted to uracil. When the modified strand is copied, through, for example, extension of a locus specific primer, a random or degenerate primer or a primer to an adaptor, a G will be incorporated in the interrogation position (opposite the C being interrogated) if the C was methylated and an A will be incorporated in the interrogation position if the C was unmethylated. When the double stranded extension product is amplified those C's that were converted to U's and resulted in incorporation of A in the extended primer will be replaced by T's during amplification. Those C's that were not modified and resulted in the incorporation of G will remain as C.

Kits for DNA bisulfite modification are commercially available from, for example, Human Genetic Signatures' Methyleasy and Chemicon's CpGenome Modification Kit. See also, W004096825A1, which describes bisulfite modification methods and Olek *et al. Nuc. Acids Res.* 24:5064-6 (1994), which discloses methods of performing bisulfite treatment and subsequent amplification on material embedded in agarose beads. In one aspect a catalyst such as diethylenetriamine may be used in conjunction with bisulfite treatment, see Komiyama and Oshima, *Tetrahedron Letters* 35:8185-8188 (1994). Diethylenetriamine has been shown to catalyze bisulfite ion-induced deamination of 2'-deoxycytidine to 2'-deoxyuridine at pH 5 efficiently. Other catalysts include ammonia, ethylene-diamine, 3,3'-diaminodipropylamine, and spermine. In some aspects deamination is performed using sodium bisulfite solutions of 3-5 M with an incubation period of 12-16 hours at about 50°C. A faster procedure has also been reported using 9-10 M bisulfite pH 5.4 for about 10 minutes at 90°C, see Hayatsu *et al, Proc. Jpn. Acad.* Ser. B 80:189-194 (2004).

Bisulfite treatment allows the methylation status of cytosines to be detected by a variety of methods. For example, any method that may be used to detect a SNP may be used, for examples, see Syvanen, *Nature Rev. Gen.* 2:930-942 (2001). Methods such as single base extension (SBE) may be used or hybridization of sequence specific probes similar to allele specific hybridization methods.

In one aspect the DNA sample is fragmented with one or more restriction enzymes and ligated to one or more adaptor sequences before treatment with bisulfite. The bisulfite treated sample may then be amplified by PCR using primers that are complementary to the adaptors. The conditions of the amplification may be selected to preferentially amplify fragments of a selected size, for example, 200 to 2000 bp, to reduce the complexity of the sample.

In one aspect adaptors are ligated to the DNA after bisulfite treatment. In a preferred aspect T4 RNA ligase is used for ligation of adaptors. For addition of PCR primers, a primer may be ligated to the 5' end (preferably the primer is end protected), then the 3' end of the fragments may be dephosphorylated and a 5' phosophorylated primer may be ligated to the 3' end. The bisulfite treatment may degrade the DNA so adaptors that are ligated before bisulfite treatment may be damaged or cleaved off by the treatment, making the fragments resistant to amplification. Bisulfite treatment may also make the DNA single stranded so T4 RNA ligase may be necessary to attach adaptor sequences. Bisulfite treatment also introduced mismatches in the DNA. Conversion of unmethylated cytosines to uracils results in G:U basepairs in place of G:C base pairs and can result in denaturation of double stranded DNA.

In addition to deamination of unmethylated cytosines, bisulfite treatment can result in damage to the DNA that results in fragmentation of the DNA. In some aspects the bisulfite treatment requires long (~4-16 hour) incubations at a pH of about 5. During this step cytosines are sulfonated and then deamination occurs. This step also may have the unintended side effect of partial depurination of the DNA. Following deamination the sulfate groups are removed by an alkali treatment. The alkali treatment may result in strand breaks at sites where depurination has occurred. The resulting fragments can be ligated to adaptors, but it may be necessary to treat the fragments chemically or enzymatically to generate ends suitable for ligation. In some aspects alkaline hydrolysis of a depurinated site may result in a 5' phorphorylated end that is suitable for ligation of an adaptor and a 3' end that is not a suitable substrate for ligation because it lacks a 3' OH. The 3' end may be treated to remove modifications that would block ligation. In one aspect the fragments are treated with an AP endonuclease prior to ligation of adaptors. In another aspect the adaptor may be ligated to the fragments in a first reaction to ligate adaptors to the ends that are available for ligation, the reaction may then be treated, for example, with kinase to remove phosphates from 3' ends, and subjected to a second ligation reaction. The ends that result after depurination and chain breakage may vary depending on the mechanism of cleavage. In some aspects a 3' phosophorylated ribose is generated, but in some aspects a mixture of ends are generated including fragments with a terminal ribose. In preferred aspects the 3' end is chemically or enzymatically processed to create an end that is suitable for adapter ligation.

In another aspect amplification of bisulfite treated DNA may be primed with random primers, for example, random hexamers. Other methods of amplification may also be used, for example, isothermal strand displacement amplification, rolling circle amplification (Lizardi *et al*., *Nat. Genet.* 19:225-232 ,1998), multiple displacement amplification (Dean *et al., Proc. Natl. Acad. Sci.* 99:5261-5266, 2002) and methods such as those described in US Patent Pub Nos. 20040209298 and 20040209299. Bisulfite treatment damages the DNA and the damaged DNA may amplify poorly. Amplification methods that enable amplification of degraded samples such as those obtained from Formalin-fixed, paraffin-embedded (FFPE) samples may be used to amplify bisulfite treated DNA. Amplification methods that may be preferred for degraded samples include those methods disclosed in US Patent Pub Nos. 20040209298 and 20040209299, Wang *et al*., *Gen. Res.* 14:2357-2366, 2004 and Wang *et al*., *Nuc. Acids Res.* 32:e76, 2004. In a preferred aspect, the primers used for amplification are biased for bisulfite converted DNA which will have a reduced number of G/C base pairs. In the first round of amplification unmethylated cytosine will generally have been converted to uracil so the primers may be biased to have fewer or no Gs.

In one aspect bisulfite treated DNA is incubated with antibodies to 5-meC or with 5-meC binding proteins and antibodies to the proteins and antibody associated complexes are isolated. The DNA from the isolated complexes may be amplified by adaptor ligation and PCR amplification as described above.

In another aspect activation-induced cytidine deaminase (AID) is used as an alternative to bisulfite treatment. AID deaminates unmethylated cytosines while methylated-CpG motifs are protected from AID-mediated deamination, see, Larijani *et al., Mol Immunol.* 42(5):599-604 (2005). AID treated DNA may be analyzed by the same methods bisulfite DNA is analyzed. The AID assay had the advantage that it can be performed in a short time, about 30 minutes compared to more than 12 hours for a typical bisulfite treatment, there are fewer steps than the complicated bisulfite treatment, and fewer toxic chemicals are used. In some aspects DNA may be treated with a combination of AID treatment and bisulfite treatment. This combined approach of the two methods may be used to improve the efficiency of the AID treatment but provide for shorter bisulfite treatment and reduced degradation of the DNA.

In one aspect the methylation level of a specific cytosine may be quantified. The hybridization pattern may be analyzed to measure the levels of methylation, hybridization intensity correlating with degree of methylation. For example, if a particular cytosine is methylated in 80% of the DNA in the sample the normalized intensity of the C "allele" should be about 4 fold the normalized intensity of the T "allele" after bisulfite treatment. Methods for quantifying methylation levels of specific cytosines using bisulfite treatment have been disclosed, for example, in Thomassin *et al., Nuc. Acids Res.* 32:e168 (2004).

In a preferred aspect the products are analyzed by hybridization to an array. In one exemplary embodiment an array is designed to detect the products of bisulfite modification using the same principles as the commercially available Affymetrix 10K Mapping Array. The 10K array has probe sets for each of more than 11,000 different human SNPs. Each probe set has a first plurality of probes that are perfectly complementary to a first allele of the SNP and a second plurality of probes that are perfectly complementary to the second allele of the SNP. If the first allele is present signal is detected by the first plurality of probes and if the second allele is present signal is detected by the second plurality of probes. Heterozygotes result in signal detection by both. The probe sets may include control probes, for example, mismatch probes, probes that shift the interrogation position relative to the central position of the probe may be included, for example, the SNP position may be at the central position or it may be shifted 1 or more positions 5' or 3' of the center of the probe. Analogous probe sets could be designed for suspected sites of methylation, treating the position as though it were a SNP with alleles C/G or T/A. Both strands may be analyzed. Exemplary probes and arrays are described in US patent application No. 10/681,773 and US Patent Nos. 5,733,729, 6,300,063, 6,586,186, and 6,361,947. The bisulfite treatment can modify any unmethylated C in the fragments, including C's in primer binding sites and C's that are in regions surrounding an interrogation positions. In preferred embodiments the adaptors are designed to take this into account, for example, the adaptor may be designed so that there are no C's in the primer binding site, the primer may also be synthesized with modified bases that are resistant to bisulfite modification so that the sequence of the primer binding site is not changed by the treatment, for example, C's could be methylated, or the primer can be designed assuming that the C's in the adaptor will be changed to U's.

Resequencing arrays which allow detection of novel SNPs from a sequence may also be used to detect the products of the bisulfite treatment. Resequencing arrays and resequencing methods are described, for example, in Cutler *et al. Genome Res.* 2001 Nov;11(11):1913-25 and in US patent publication No. 20030124539, both of which are incorporated herein by reference in their entirety. In general resequencing arrays detect all possible single nucleotide variations in a reference sequence. Probes are included that are perfectly complementary to the reference sequence and interrogate a plurality of positions in the sequence individually for variation in the reference sequence. Probes that are perfectly complementary to the variant sequence are included for each possible variation. An array may be tiled to detect all possible single nucleotide variations in one or more reference sequences. The reference sequence or sequences interrogated by the array may be, for example, one or more entire chromosomes, one or more entire genomes, one or more mitochondrial genomes, or selected regions of interest from within one or more genomes. In one embodiment a resequencing array is tiled with regions that are known or suspected to be methylated. In some embodiments CpG sites may be close together so that the probes of the array may be complementary to overlapping CpG sites. For example if the probe is a 25 mer and the interrogation position at position 13 is complementary to a first cytosine position there may be a second CpG that is within the 12 base pairs upstream or the 12 base pairs downstream of the first cytosine. The second cytosine may or may not be methylated. Probes can be designed to detect both possibilities, i.e. both methylated (both C), both unmethylated (both T), one methylated (C) and the other unmethylated (T). Probes that are perfectly complementary to each possible outcome may be designed.

In another aspect of the invention amplified methylated target is enriched relative to unmethylated target. In one exemplary embodiment a nucleic acid sample suspected of containing m5C is fragmented using a restriction enzyme and adaptors are ligated to the fragments. Antibodies to m5C are used to isolate adaptor-ligated fragments that contain m5C. Alternatively the nucleic acid may be incubated with proteins that specifically bind m5C and then antibodies to those proteins may be used to isolate methylated fragments. Antibodies to 5-meC are available, for example, ab1884 available from Abcam (Cambridge, UK). The isolated fragments are amplified by PCR using a primer complementary to the adaptor and the amplified fragments may be hybridized to an array of probes. In a preferred aspect the probes of the array are complementary to one or more regions of the genome. Regions of the array that show hybridization above background are indicative of areas of the genome that are methylated. In a preferred embodiment the array comprises probes to CpG rich regions of the genome, intragenic regions, or regions known or predicted to be regulatory regions. In another embodiment the immunoprecipitated fragments are treated with bisulfite so that precise locations of methylated cytosines may be identified. The sample may be analyzed by hybridization to an array of sequence specific probes as described above.

In one aspect of the invention methyl binding proteins, such as MeCP2 and SAP 18/30 (Sin3 associated Polypeptides 18/30), are mixed with the genomic DNA sample and used to enrich for methylated sequences. Antibodies to methyl CpG binding domain proteins (MBDs), for example, MBD2 and MBD3 may be used to isolate DNA containing methylation. Antibodies against 5-meC-binding proteins are available, for example, antibodies to MeCP2 (IMG-297) are available from Imgenex Corp. (San Diego, CA). In another aspect antibodies that recognize 5-meC may be used to enrich for methylated sequences. The DNA is preferably denatured prior to antibody binding.

In another aspect of the invention methylation is used as a means of separating a genome into subsets in a relatively reproducible manner in order to reduce the complexity of the sample prior to further analysis. Some regions of the genome are stably methylated while other regions are stably unmethylated. Mechanisms that differentiate between methylated and unmethylated DNA can be used to obtain fractions of a sample that are enriched for either methylated or unmethylated DNA. In this way the complexity of a sample can be reduced. Separation may be prior to if amplification is by a method that maintains methylation information. It is often desirable to reduce the complexity of a sample that contains a complex mixture of nucleic acids prior to hybridization to improve sensitivity of detection and minimize background.

In one aspect of the invention methods for analyzing nucleic acid samples following separation of methylated and unmethylated fractions are disclosed. The fraction that is analyzed may be the methylated or unmethylated fraction or a comparison of methylated and unmethylated fractions may be made. In many embodiments the unmethylated fraction is enriched, for example through separation of methylated DNA from unmethylated or by preferential amplification of unmethylated DNA. Isolation of a fraction that is enriched for a subset of the starting nucleic acids may be used as a method of reducing the complexity of a sample or as a method of measuring differences between the methylated fraction and the unmethylated fraction. In one embodiment the methods are particularly useful for analyzing a sample to identify regions of the genome that are present in the unmethylated fraction and regions of the genome that are present in the methylated fraction. In many embodiments the methods for separation of methylated and unmethylated nucleic acids are combined with methods of analysis of nucleic acids with arrays of probes.

In one aspect CpG islands are enriched by digesting the DNA sample with an enzyme, such as Msel followed by size selection. Msel cuts genomic DNA into small fragments but cuts infrequently in CpG islands. The larger fragments, enriched for CpG islands, may be separated from the smaller fragments by any available size separation method.

Reduced complexity samples that are a representation of a more complex sample, such as a genome can be used for a variety of analysis methods, including those that involve hybridization. Reduced complexity samples may be used, for example, for sequencing applications, genotyping, quantitative assessment of copy number, LOH analysis, and CGH analysis. In many embodiments the analysis is by hybridization of the reduced complexity sample to an array of probes. Arrays for expression analysis, resequencing, and genotyping, for example, are available from Affymetrix, Inc., Santa Clara, CA.

Methods for separation of methylated from unmethylated nucleic acids have been described, see, for example, US patent publication nos. 20010046669, 20030157546, and 20030180775 which are each incorporated herein by reference in their entireties.

Repetitive sequences in plant and mammalian genomes are often present in high copy number, have high levels of cytosine and low transcriptional activity (See, e.g., Martienssen, R. A. (1998) Trends Genet. 14:263; Kass, S. U., et al. (1997) Trends Genet. 13:335; SanMiguel, P., et al., (1996) Science 274:765; Timmermans, M. C., et al. (1996) Genetics 143:1771; Martienssen, R. A. and E. J. Richards, (1995) Curr. Opin. Genet. Dev. 5:234-242; Bennetzen, J. L., et al. (1994) Genome 37:565; White, L. F., et al. (1994) Proc. Natl. Acad. Sci. U.S.A. 91:11792; Moore, G., et al. Genomics 15:472). High copy DNA sequences are frequently methylated and often are not present in areas of expressed genes. Methods that eliminate or reduce the representation of such high copy methylated DNA from a library or from a nucleic acid sample may be used to enrich for target sequences of interest and result in a sample that has a complexity that is reduced, facilitating further analysis. Often the unmethylated regions are the regions that contain the genes and are of the highest interest for analysis.

Nucleic acid samples may be enriched for sequences that are unmethylated by propagation of nucleic acid libraries, for example genomic libraries which may be partial libraries, in methylation restrictive hosts, such as *E. coli* strains JM101, JM107 and JM109. This method, methylation filtration, was recently used to sequence the genome of maize, see Palmer et al. *Science* 302:2115-2117 (2003). The method prevents the propagation of clones carrying methylated inserts, resulting in the enrichment of genes five to sevenfold when compared to control libraries.

In another embodiment nucleic acid samples are digested with enzymes that are methylation sensitive, for example enzymes that cleave only unmethylated DNA or cleave only methylated DNA or methylation insensitive enzymes that cleave methylated or unmethylated DNA. Differentially digested samples may be amplified and the amplified fragments may be labeled and then detected using microarrays. A sample may be digested in parallel with a methylation sensitive enzyme and a methylation insensitive enzyme and analyzed to determine which sequences are present following each treatment. Sequences that are present in the first sample but not the second sample indicate that the sequence was methylated.

In one exemplary embodiment a nucleic acid sample is obtained from a source, such as from an individual, the nucleic acid may be fragmented, for example by digestion with one or more restriction enzymes, and an adaptor sequence may be attached to the fragments to generate adaptor-ligated fragments. The adaptor-ligated fragments may be digested with an enzyme that cleaves methylated DNA but not unmethylated DNA, for example, McrBC. The sample may then be amplified with a primer that hybridizes to the adaptor sequence. The methylated fragments that have been cut with the methyl specific enzyme are not amplified because they have the adaptor only on one end, resulting in selective amplification of unmethylated DNA.

The amplified products may be detected by, for example, hybridization to a microarray. The McrBC digested sample may be compared with a parallel sample that was not digested with McrBC to identify regions that were methylated. If the products are hybridized to an array of probes in parallel, probes to the regions that were methylated in the sample should show hybridization in the sample that was not digested with McrBC but not in the sample that was digested with McrBC. Because the presence of methylation in the fragment is detected by detecting the presence or absence of the restriction fragment there is considerable flexibility in the design of the probes that would be suitable. For example, the fragments to be detected will typically be between 200 and 1,000 base pairs and probes may be targeted to any region of the fragment. Probes need not be complementary to the site of methylation but can be complementary to a site upstream or downstream. Probes may be targeted to one region of the fragment or a plurality of regions in the fragment, they may be targeted to a specific feature of the fragment, for example, a SNP in the fragment or to one or more CpG's in the fragment. In one embodiment an array of probes comprising probes spaced evenly throughout the genome may be used.

In an exemplary embodiment the amplified products are labeled and hybridized to a genotyping array, for example, the Mapping 10K or 100K Array (Affymetrix, Santa Clara). The GeneChip Mapping Assay (WGSA) may be used to reduce the complexity of a sample. The basic steps of the assay are as follows: total genomic DNA (250ng) is digested with a restriction enzyme (e.g. XbaI) and ligated to adaptors that recognize the cohesive four basepair overhangs. All fragments resulting from restriction enzyme digestion, regardless of size, are substrates for adaptor ligation. A generic primer that recognizes the adaptor sequence is used to amplify adaptor ligated DNA fragments. PCR conditions that are optimized to preferentially amplify fragments of a selected size range (e.g. 250 to 2000 bp) are used for amplification. Conditions may be optimized to select for different size ranges, for example 200 to 1,000 base pairs. The amplified DNA is then fragmented, labeled and hybridized to the Mapping 10K Array. The probes of the array are selected to be complementary to regions of the genome that are predicted by in silico digestion to be present on fragments of the selected size range (e.g. 250 to 1000 bp when the genome is digested with Xbal). In this way the amplification enriches for a subset of fragments, the same subset of fragments is reproducibly enriched and the array is designed to interrogate at least some of those fragments. The Mapping 10K and 100K Array interrogates the genotype of known SNPs present on the predicted fragments, but in other embodiments an array may be designed to interrogate for the presence or absence of a fragment. For additional information about the Mapping 10K array and assay see the GeneChip Human Mapping 10K Array and Assay Kit Data Sheet, part no. 701366 Rev. 4, Affymetrix, Inc. and the Mapping 10K Manual.

In one embodiment arrays that comprise probes that are complementary to genes in an organism, may be used to analyze methylated or unmethylated fractions. For example, expression arrays available from Affymetrix, such as the Human Genome U133 Plus 2.0 array, may be used. Expression arrays are available for a number of organisms including Mouse and Rat and can be custom designed for an organism of choice. Arrays comprising probes to predicted or known exons, or splice junctions (intron-exon or exon-exon) may also be used.

In one embodiment high density arrays that tile an entire genome, one or more entire chromosomes or a representation of an entire genome or one or more entire chromosomes may be used to analyze a sample prepared by separation of methylated and unmethylated DNA. For example, an array that contains probes spaced on average every 35 base pairs along one or more chromosomes or an entire genome may be used. See, for example, Kapranov et al. Science 296:916-919 (2002). See also US Patent Application Nos. 10/741,193, 10/736,054, 10/714,253, and 10/712,322. In one embodiment a sample that has been enriched for unmethylated sequences may be analyzed by transcription factor binding affinity. Sequences that bind to transcription factors may be purified by affinity to transcription factors and then identified by array analysis. Complexity may similarly be reduced by enrichment for methylated sequences, by digestion with enzymes that cleave only unmethylated DNA.

A number of methyl-dependent restriction enzymes are known to those of skill in the art and are available commercially from, for example, New England Biolabs. Examples of methyl-dependent restriction enzymes include, McrBC, McrA, MrrA, and DpnI. McrBC is an endonuclease which cleaves DNA containing methylcytosine, (e.g. 5-methylcytosine or 5-hydroxymethylcytosine or N4-methylcytosine, reviewed in Raleigh, E. A. (1992) *Mol. Microbiol.* 6, 1079-1086) on one or both strands. McrBC will not act upon unmethylated DNA (Sutherland, E. et al. (1992) *J. Mol. Biol.* 225, 327-334). The recognition site for McrBC is 5' ... Pu^{m}C (N₄₀₋₃₀₀₀) Pu^{m}C ... 3'. Sites on the DNA recognized by McrBC consist of two half-sites of the form (G/A)^{m}C. These half-sites can be separated by up to 3 kb, but the optimal separation is 55-103 base pairs (Stewart, F. J. and Raleigh E. A. (1998) *Biol. Chem.* 379, 611-616 and Panne, D. et al. (1999) *J. Mol. Biol.* 290, 49-60.). McrBC requires GTP for cleavage, but in the presence of a non-hydrolyzable analog of GTP, the enzyme will bind to methylated DNA specifically, without cleavage (Stewart, F. J. et al. (2000) *J. Mol. Biol.* 298, 611-622). Recombinant McrBC is available from, for example, New England Biolabs. McrBC may be used to determine the methylation state of CpG dinucleotides. McrBC will act upon a pair of Pu^{m}CG sequence elements, but will not recognize Hpa II/Msp I sites (CCGG) in which the internal cytosine is methylated. The very short half-site consensus sequence (Pu^{m}C) allows a large proportion of the methylcytosines present to be detected.

In one embodiment reaction conditions for digestion with McrBC are 50 mM NaCl, 10 mM Tris-HCI, 10 mM MgCl₂, 1 mM dithiothreitol (pH 7.9at 25°C) with 100 pg/ml BSA and ImM GTP. Incubate at 37°C. Conditions may be varied. NEB defines one unit as the amount of enzyme required to cleave 1 µg of a plasmid containing a single McrBC site in 1 hour at 37°C in a total reaction volume of 50 µl. A 5 to 10-fold excess of enzyme may be used for cleavage of genomic DNA. The enzyme may be heat inactivated by heating to 65°C for 20 minutes. McrBC makes one cut between each pair of half-sites, cutting close to one half-site or the other, but cleavage positions are distributed over several base pairs approximately 30 base pairs from the methylated base. See also, Bird, A.P. (1986) *Nature* 321, 209-213 and Gowher, H. et al. (2000) *EMBO J.* 19, 6918-6923.

Studies on or utilizing McrBC have been reported in the literature, for example, Gast *et al. Biol Chem.* 378(9):975-82, (1997), Pieper et al., Rabinowicz, *Methods Mol Biol.* 236:21-36 (2003), Badal *et al. J Virol.* 77(11):6227-34 (2003) and Chotai and Payne, *J Med Genet.* 35(6):472-5 (1998). See also, Lyko, F. et al. *Nat. Genet.,* 23, 363-366 (2000) which used McrBC as a tool for enrichment of undermethylated DNA in drosophila.

In one aspect, genomic DNA is divided into a methylated fraction and an unmethylated fraction by any method known in the art. Each fraction may be separately hybridized to an array or each fraction may be labeled with a differentially detectable label, for example different colors of fluorescent dye (for example, unmethylated DNA may be labeled with green and methylated DNA may be labeled with red) and then both may be hybridized to the same array of probes. See for example, U.S. Patent No. 6,576,424, which is incorporated herein by reference. If a region of the genome was not methylated then the feature or features corresponding to that region of the genome will be detected as green. If the region is methylated then the feature should be detected as red. If both red and green are detected the region may have been partially methylated in the sample, a ratio of red to green may be used to determine the extent of methylation.

In one aspect the disclosed methods are used to obtain a methylation signature or profile of a tumor or tissue. Methylation is of particular interest in the diagnosis, treatment and outcome prediction for cancer, see Jones and Baylin, *Nat. Rev. Genet.* 3:415-428 (2002) and Bird, *Genes Dev.* 16:6-21 (2002). Patterns of methylation may be associated with specific tumors. Samples from a specific type of tumor may be isolated and analyzed using the methods disclosed to obtain a methylation pattern characteristic of a tumor type or the stage of a tumor. In one embodiment a sample from an individual or from a tumor may be compared to the methylation pattern of a tumor of known type or stage to determine if the unknown sample is similar to one or more of the known tumor types in methylation pattern. Patterns obtained according to the methods may be used to diagnose disease, stage disease, monitor treatment, predict treatment outcome, and monitor disease progression. In many embodiments analysis is performed by a direct comparison of a hybridization pattern without correlation of the pattern to the presence or absence of any specific sequence. Differences or similarities between a pattern obtained from an unknown sample that is being analyzed and patterns obtained from known samples can be used to determine if the unknown is likely to match the known sample in methylation pattern.

In one embodiment blood samples are analyzed to detect changes in the methylation pattern of tumor cells that are sloughed-off into the blood stream. Patterns of aberrant methylation or demethylation that are characteristic of a tumor type may be identified by analysis of a blood sample. In one exemplary embodiment the sample is fragmented with a first restriction enzyme and the fragments are ligated to adaptors. The adaptor-ligated fragments are then digested with an enzyme that is methylation dependent or methylation sensitive. The adaptor-ligated fragments that are not digested are amplified by PCR using a primer to the adaptor. The products of the PCR amplification are hybridized to an array of probes to generate a hybridization pattern. The hybridization pattern may be compared to a hybridization pattern from another sample that has been similarly treated. Differences between hybridization patterns are indicative of differences in the methylation patterns between the two samples. A data base of hybridization patterns that are characteristic of disease states, normal states, or tissue types may be generated and used to compare hybridization patterns of unknown samples to identify similar patterns. See, for example, U.S. Patent No. 6,228,575 which discloses methods of sample characterization based on comparison of hybridization pattern. A variety of arrays may be used for this purpose and it is not necessary that the array be specifically designed to detect specific genomic sequences from the organism being analyzed.

In one embodiment enrichment of unmethylated DNA is combined with comparative genomic hybridization (CGH) to analyze tumor cells to identify differences between tumor DNA and normal DNA. See, for example, Kallioniemi et al. Methods 9(1):113-121 (1996). Equal amounts of differentially labeled tumor DNA and normal reference DNA, (one may be labeled with biotin and the other with digoxigenin, for example), may be hybridized to an array of probes the signal intensities are quantified and over and underrepresented in tumor versus normal can be quantified. In one embodiment methods of analysis of methylation status may be combined with methods of estimating copy number of one or more regions of a genome. Many cancers are associated with increases in the copy number of one or more regions of the genome. Increased copy number can be detected by hybridization to arrays. The increase of copy number is detected as an increase in the intensity of hybridization. Methods for analysis of copy number using oligonucleotide arrays are disclosed, for example, in U.S. Patent Pub. No. 20040157243 which discloses specific computer methods to perform copy number analysis using, for example, the Affymetrix 10K Mapping Array and Assay.

In another aspect method of complexity reduction that employ separation of fractions based on the presence or absence of methylation are used to enrich for sequences of interest in a sample that is a mixture of host and a pathogen genomic DNA. Some organisms lack 5-meC modifications in their genomes or have reduced levels of 5-meC. For example, pathogens such as mycoplasma have an absence of 5-meC or very low levels. For additional examples see, for example, Razin and Razin, *NAR* 8:1383-1390 (1980). The unmethylated pathogen DNA may be enriched by digesting the sample with a methyl dependent enzyme such as McrBC. Unmethylated pathogen DNA may also be enriched by depletion of methylated DNA using antibodies to 5-meC or 5-meC binding proteins in combination with antibodies to the binding proteins. In one aspect the sample is first fragmented with a restriction enzyme that does not have CpG in its recognition site and adaptors are ligated to the fragments. The adaptor-ligated fragments are digested with a methylation dependent enzyme so fragments that are methylated and contain the enzyme recognition site are fragmented. The adaptor-ligated fragments that were not fragmented by the methylation dependent enzyme are amplified by PCR using a primer to the adaptor. This results in an amplification product that is enriched for unmethylated DNA relative to methylated DNA.

In one embodiment methods of reducing the complexity of a genomic sample using methods that result in preferential amplification of unmethylated nucleic acids may be used to enrich for pathogen DNA in a complex mixture. For example, if a nucleic acid sample is isolated from a patient who is thought to be infected with a pathogen, the nucleic acid sample may contain a mixture of the patient's DNA and the pathogen's DNA. Many prokaryotic pathogens have lower levels of methylation than the organisms that they infect so treating the mixed sample with enzymes that preferentially degrade methylated DNA prior to amplification may be used to enrich the pathogen DNA relative to the host DNA. The amplified sample may then be analyzed to detect the pathogen DNA by, for example, hybridization to an array of nucleic acid probes. Potential interfering effects due to the presence of the host DNA are reduced allowing for improved detection of the pathogen DNA.

In one embodiment a nucleic acid sample that is suspected of containing pathogen DNA is fragmented to produce fragments and adaptors are attached to the ends of the fragments. The adaptor modified fragments are then treated with an enzyme that cleaves methylated DNA but not unmethylated DNA, for example McrBC. Fragments that contain a recognition site for McrBC will be cleaved into smaller fragments that have the adaptor sequence on only one end. The sample is then amplified by PCR using a primer or primers that are complementary to sequence in the adaptors. Fragments that were cleaved by McrBC will not be amplified because they have an adaptor and therefore a priming site at only one end. Because the pathogen sequence is not methylated it will not be cleaved by McrBC and will be amplified.

Arrays that may be used for detection of methylation include, for example, tiled arrays, arrays that have probes that are perfectly complementary to a plurality of possible combinations of CpG and 5-meCpG after bisulfite treatment for the region of interest. Methylation may be analyzed on both strands or on one strand. If probes are designed to one strand they may be designed to interrogate either strand. Choice of strand to be interrogated in some aspects is the strand containing the cytosine, while in other aspects, that strand has been amplified after modification so that the resulting amplified double stranded product has an A:T basepair in place of the C:G base pair and either strand can be interrogated. All unmethylated cytosines may be converted to uracils and probes and primers may be designed to take this into account, for example, probe locations that are complementary to positions that are cytosines in the genomic sequence should have A's in the position that is complementary to the cytosine position.

There are estimated to be about 28 to 29 million CpG's in the human genome and the density is predicted to be about 1 CpG every 50-100 base pairs in low density CpG regions and about 1 CpG every 20 base pairs in high density regions . In one aspect an array is designed to interrogate methylation status of more than 50,000, more than 100,000, more than 500,000, more than 1,000,000, more than 2,500,000 or more than 5,000,000 of these CpG's. In some embodiments the array may also contain probes to interrogate CNG positions which can also be methylated at the cytosine. Interrogation may be, for example, analogous to detecting a polymorphism at the cytosine position, reflecting the change of the cytosine to a uracil by either chemical, for example bisulfite, or enzymatic, for example AID, mechanisms. Particular CpG's s may be selected for interrogation based on the positioning of neighboring CpG dinucleotides. When there are more than one CpG in the region that the probe is complementary to, for example, within the 25 bases of the probe, the perfect complementarity of the probe to interrogate the central CpG may be impacted by the methylation status of the second, third or fourth CpG within the probe region. In some aspects the probe set for interrogation of the first CpG (the interrogation CpG) may be designed to take in all possible combinations of sequence variation resulting from variation in the methylation status of the secondary (non-interrogation) CpGs. This would require additional probes for each possible sequence variation. In another aspect CpGs that do not have another CpG within 12, 15, 20 or 30 bases upstream or downstream are selected for interrogation.

### CONCLUSION

Methods of amplifying genomic DNA that preserve epigenetic information are disclosed. In one aspect a DNA methyltransferase activity is included during amplification of genomic DNA. The methytransferase activity recognizes hemimethylated sites and methylates newly synthesized DNA during amplification using the methylated template DNA as a guide.

The above description is illustrative and not restrictive. Many variations of the invention will become apparent to those of skill in the art upon review of this disclosure. The scope of the invention should, therefore, be determined not with reference to the above description, but instead be determined with reference to the appended claims along with their full scope of equivalents.

## Claims

1. A method for amplifying genomic DNA wherein methylation status is maintained, comprising:
(a) obtaining a sample comprising methylated genomic DNA;
(b) hybridizing one or more primers to the genomic DNA;
(c) extending the one or more primers with a DNA polymerase to generate hemimethylated hybrids comprising a newly synthesized unmethylated cDNA strand and a methylated template strand;
(d) treating the hybrids with a DNA methyltransferase activity in the presence of a methyl donor, wherein the DNA methyltransferase activity methylates hemimethylated sites in double stranded DNA, to generate methylated hybrids comprising a newly methylated cDNA strand and a methylated template strand;
(e) denaturing the methylated hybrids; and
(f) hybridizing one or more primers to the products of step (e) and repeating steps (c) and (d) at least once to generate a methylated, amplified product.

2. The method of claim 1 wherein the DNA polymerase is a thermal stable polymerase.

3. The method of claim 1 wherein the one or more primers comprise a collection of random sequence primers.

4. The method of claim 1 wherein the one or more primers comprise a plurality of locus specific primers that are perfectly complementary to a region of at least 15 bases that is within 1,000 bases of a CpG island.

5. The method of claim 1 wherein the DNA polymerase is a strand displacing enzyme.

6. The method of claim 5 wherein the strand displacing enzyme is phi29.

7. The method of claim 1 wherein the DNA methyltranferase activity comprises a Dnmtl l enzyme.

8. The method of claim 7 wherein the Dnmtl is selected from the group consisting of mouse Dnmt 1 and human Dnmt1.

9. The method of claim 7 wherein the Dnmt1 is recombinant.

10. The method of claim 1 wherein the methyl donor is S-adenosylmethionine.

11. The method of claim 1 wherein the DNA methyltransferases activity is a variant form of human or mouse Dnmt1 that has increased specificity for hemimethylated DNA relative to the native enzyme.

12. A method for analyzing the methylation status of at least one genomic region in a genomic DNA sample to identify regions that are methylated, comprising:
(a) amplifying the genomic DNA sample according to the method of claim 1 to generate an amplified sample comprising amplified, methylated products;
(b) fragmenting the amplified sample from (a) with a first restriction enzyme and ligating adaptors to the fragments to generate adaptor-ligated fragments;
(c) treating a first aliquot of sample from (b) with a second restriction enzyme and a second aliquot of the sample from (b) with a third restriction enzyme, wherein the second and third restriction enzymes are isoshizomers, wherein the second restriction enzyme is methylation sensitive and the third restriction enzyme is methylation insensitive;
(d) amplifying the first and second aliquots from (c) by PCR using a primer complementary to the adaptor to generate a first amplified sample from the first aliquot and a second amplified sample from the second aliquot;
(e) labeling the products of (d) and hybridizing the labeled products in parallel to an array of probes to generate a first hybridization pattern from the first amplified sample and a second hybridization pattern from the second amplified sample, wherein the array comprises a plurality of probes that are each at least 15 bases and are each perfectly complementary to a restriction fragment that is between 200 and 2,000 base pairs from the genome of a selected organism when the genome of the selected organism is digested with said first restriction enzyme; and
(f) comparing the first and second hybridization patterns to identify fragments that are detected as present in the first pattern and absent from the second pattern, wherein those fragments that are present in the first pattern are identified as methylated regions.

13. The method of claim 1 further comprising analyzing the methylation status of a plurality of genomic regions in the amplified sample to identify a plurality of regions that are not methylated, by a method comprising:
fragmenting the amplified sample to produce fragments;
ligating an adaptor to the fragments to generate a sample comprising adaptor-ligated fragments;
dividing the sample comprising adaptor-ligated fragments into at least a first and a second aliquot;
treating the first aliquot with a methylation insensitive restriction enzyme;
treating the second aliquot with a methylation dependent restriction enzyme;
amplifying the treated first and second aliquots using a primer complementary to the adaptor;
labeling the amplified products and hybridizing the amplified product to an array of probes comprising probes complementary to known genomic regions to generate a first hybridization pattern for said first aliquot and a second hybridization patter for said second aliquot;
comparing the first and second hybridization patterns and identifying unmethylated genomic regions by identifying genomic fragments that are present in the second aliquot and absent in the first aliquot.

14. The method of claim 12 wherein the first enzyme has a recognition site that does not comprise CpG.

15. The method of claim 12 wherein the first enzyme is selected from the group consisting of XbaI, HindIII, and BglII.

16. The method of claim 12 wherein the second restriction enzyme is HpaII and the third restriction enzyme is Mspl.

17. The method of claim 13 wherein the methylation dependent enzyme is McrBC.

18. The method of claim 12 wherein the array of probes comprises at least 100,000 different probes attached to a solid support, wherein the location of the probes is determined or determinable, and wherein the probes of the array are selected by a computer system, wherein the computer system selects probes for the array by a method comprising:
modeling the fragmentation of the amplified sample to generate a first list of fragments resulting from the fragmentation, wherein the first list includes the predicted length of the fragments;
generating a second list of fragments from the first list by identifying fragments that are between 200 and 2,000 base pairs in length;
generating a third list of fragments from the second list by identifying fragments that comprise a recognition site for the second and third restriction enzymes; and
selecting probes that are complementary to a plurality of fragments in the third list.

19. The method of claim 13 wherein the array of probes comprises at least 100,000 different probes attached to a solid support, wherein the location of the probes is determined or determinable, and wherein the probes of the array are selected by a computer system, wherein the computer system selects probes for the array by a method comprising:
modeling the fragmentation of the amplified sample to generate a first list of fragments resulting from the fragmentation, wherein the first list includes the predicted length of the fragments;
generating a second list of fragments from the first list by identifying fragments that are between 200 and 2,000 base pairs in length;
generating a third list of fragments from said second list by identifying fragments that comprise a recognition site for the methylation dependent enzyme; and
selecting probes that are complementary to a plurality of fragments in the third list.

20. The method of claim 18 wherein the solid support is a plurality of beads.

21. The method of claim 19 wherein the solid support is a plurality of beads.

22. The method of claim 1 further comprising analyzing the amplified sample to identify a plurality of cytosines that were methylated in the genomic sample and a plurality of cytosines that were not methylated in the genomic sample by a method comprising converting unmethylated cytosines in the amplified sample to uracils by a method that does not convert methylated cytosines to uracils and determining the sequence that is present at a plurality of cytosine positions.

23. The method of claim 22 wherein said converting step is selected from bisulfite treatment or treatment with a cytidine deaminase.

24. The method of claim 22 wherein said converting step comprises bisulfite treatment and treatment with a cytidine daminase.

25. The method of claim 23 or 24 wherein the cytidine deaminase is an activation-induced cytidine deaminase.

26. The method of claim 22, 23, 24 or 25 wherein said determining step comprises labeling the amplified sample and hybridizing the labeled sample to an array of probes wherein the array of probes comprises probes to interrogate the sequence of a plurality of cytosines to determine if the cytosine being interrogated was methylated in the genomic sample.

27. The method of claim 26 wherein the cytosines to be interrogated are part of a CpG dinucleotide.

28. The method of claim 27 wherein the array interrogates the methylation of at least 10,000 cytosines.

29. An array of probes comprising at least 100,000 different probes attached to a solid support, wherein the location of the probes is determined or determinable, and wherein the probes of the array are selected by a computer system, wherein the computer system selects probes for the array by a method comprising:
modeling the fragmentation of a first nucleic acid sample by a first restriction enzyme to generate a first list of fragments resulting from the fragmentation, wherein the first list includes the predicted length of the fragments;
generating a second list of fragments from the first list by identifying fragments that are within a selected size range;
generating a third list of fragments from the second list by identifying fragments in the second list that comprise a recognition site for a second restriction enzyme wherein said second restriction enzyme is a methylation dependent restriction enzyme or a methylation sensitive restriction enzyme; and
selecting at least 100,000 different probes for the array wherein each probe is at least 15 5 bases and is perfectly complementary to a fragments in the third list.

30. The array of claim 29 wherein the first restriction enzyme is a combination of two or more restriction enzymes.

31. The array of claim 29 wherein the second restriction enzyme is McrBC.

32. The method of claim 29 wherein the second restriction enzyme is HpaII.

33. A method for analyzing the methylation of a plurality of different of CpG sites in a first nucleic acid sample comprising genomic DNA comprising
(a) amplifying the genomic DNA sample according to the method of claim 1 to generate a first amplified sample comprising amplified, methylated products;
(b) fragmenting the first amplified sample with a first restriction enzyme and ligating adaptors to the fragments to generate a second sample comprising adaptor-ligated fragments;
(c) treating the second sample with sodium bisulfite to generate a third sample;
(d) amplifying at least some of the adaptor-ligated fragments in the third sample by PCR using a primer complementary to the adaptor;
(e) fragmenting the products of step (d) and end labeling the fragments;
(f) hybridizing the labeled products to an array of probes to generate a hybridization pattern, wherein the array comprises a plurality of probe pairs, wherein each probe pair comprises a first probe that is complementary to a first CpG site after sodium bisulfite treatment if the C is methylated and the second probe is complementary to the same region if the C is unmethylated; and
(f) analyzing the hybridization pattern to determine, for each of a plurality of CpG sites, if the site was methylated in the first nucleic acid sample.

34. A method of reducing the complexity of a first nucleic acid sample comprising methylated genomic DNA to generate a reduced complexity sample comprising:
fragmenting the first nucleic acid sample with a first restriction endonuclease to produce a second nucleic acid sample comprising restriction fragments;
ligating an adaptor to the restriction fragments in the second nucleic acid sample to generate a third nucleic acid sample comprising adaptor-ligated fragments;
fragmenting the third nucleic acid sample with a methylation dependent endonuclease to generate a fourth nucleic acid sample; and
amplifying the fourth nucleic acid sample by PCR with a primer that is complementary to the adaptor to generate a reduced complexity sample.

35. The method of claim 34 wherein the methylation dependent enzyme is McrBC.

36. The method of claim 34 wherein the first nucleic acid sample is from the group consisting of a blood sample, a tissue sample and a tumor sample.

37. A method of obtaining a hybridization pattern characteristic of a sample comprising:
obtaining a genomic DNA sample from said sample;
reducing the complexity of the nucleic acid sample according to the method of claim 34;
fragmenting the reduced complexity sample and labeling the fragments with a detectable label; and
hybridizing the labeled fragments to an array of nucleic acid probes to obtain a hybridization pattern.

38. A method of comparing an unknown nucleic acid sample to a known nucleic acid sample comprising:
generating a first hybridization pattern for said unknown sample according to the method of claim 30;
obtaining a second hybridization pattern for said known sample, wherein the second hybridization pattern was generated according to the method of claim 37; and
comparing the first hybridization pattern to the second hybridization pattern.

39. A method of classifying a tumor into a known class of tumors comprising:
generating a first hybridization pattern for said unknown sample according to the method of claim 37;
obtaining a plurality of second hybridization patterns from a plurality of tumors of known class, wherein the second hybridization patterns were each generated according to the method of claim 37 from a sample from a tumor of known class;
comparing the first hybridization pattern to each of the second hybridization patterns to identify the second hybridization pattern that most closely matches the first hybridization pattern; and
classifying the tumor in the class of the known tumor with the most closely matching hybridization pattern.

40. A method of detecting methylated genomic regions in a genomic DNA sample comprising the following steps:
a. treating the genomic DNA sample with bisulfite;
b. fragmenting the genomic DNA sample;
c. ligating an adaptor to the fragments;
d. amplifying the adaptor-ligated fragments;
e. labeling the amplified fragments with a detectable label and hybridizing the labeled fragments to an array to generate a hybridization pattern; and
f. comparing the hybridization pattern to a reference to identify methylated genomic regions.

41. The method of claim 40 wherein step (a) is performed before step (b) and wherein step (c) comprises ligating an adapter to the 5' end of fragments using an RNA ligase, removing 3' phosphates and ligating adaptors to the 3' ends of fragments.

42. The method of claim 40 further comprising obtaining a sample that is enriched for fragments containing 5 methyl cytosine by incubating the bisulfite treated sample with an antibody to 5 methyl cytosine or with a protein that binds 5 methyl cytosine and an antibody to said protein and isolating antibody complexes, wherein said isolating step is performed prior to step (c).

43. The method of claim 40 wherein step (b) is performed before step (a).

44. The method of claim 40 wherein the bisulfite treatment comprises incubation with 8 to 10 M bisulfite for between 5 minutes and 1 hour.

45. A method for analyzing the methylation status of one or more cytosines in a nucleic acid sample, said method comprising: amplifying at least some sequences in the nucleic acid sample, wherein the methylation pattern of at least some of the sequences in the starting nucleic acid sample is copied during the amplification step to generate a methylated amplified sample; subjecting the methylated amplified sample to a treatment that differentially modifies methylated cytosines and unmethylated cytosines; and detecting the methylation status of at least one cytosine in the amplified sample by hybridization to an array of nucleic acid probes.

46. The method of claim 45 wherein said treatment that differentially modifies methylated cytosines and unmethylated cytosines is bisulfite treatment.

47. The method of claim 45 wherein said treatment that differentially modifies methylated cytosines and unmethylated cytosines is treatment with an activation-induced cytidine deaminase.

48. The method of claim 45 wherein treatment that differentially modifies methylated cytosines and unmethylated cytosines comprises treatment with an activation-induced cytidine deaminase and bisulfite treatment.

49. The method of claims 46, 47 or 48 wherein said array of probes comprises probes that are perfectly complementary to a plurality of different sequences that would result after bisulfite treatment or treatment with an activation-induced cytidine deaminase.

50. The method of claim 49 wherein the array of probes comprises probes that are perfectly complementary to all possible sequence combinations resulting after bisulfite treatment or treatment with an activation-induced for a plurality of selected genomic regions.
